(19) Europäisches Patentamt
European Patent Office
Office européen des brevets

(11) EP 0 715 843 B1

(12) **EUROPÄISCHE PATENTSCHRIFT**

(45) Veröffentlichungstag und Bekanntmachung des Hinweises auf die Patenterteilung:
**08.03.2000 Patentblatt 2000/10**

(51) Int Cl.7: **A61K 7/06**

(21) Anmeldenummer: **95119001.6**

(22) Anmeldetag: **02.12.1995**

(54) **Als Wirkstoffe in kosmetischen Zubereitungen wie Haarstylingmitteln geeignete quaternierte Copolymerisate**

Quaternized copolymers as agents in cosmetic preparations for hair styling aid

Copolymères quaternisés comme agents dans des compositions cosmétiques pour la mise en forme des cheveux

(84) Benannte Vertragsstaaten:
**BE CH DE ES FR GB IT LI NL**

(30) Priorität: **07.12.1994 DE 4443568**

(43) Veröffentlichungstag der Anmeldung:
**12.06.1996 Patentblatt 1996/24**

(73) Patentinhaber: **BASF AKTIENGESELLSCHAFT
67056 Ludwigshafen (DE)**

(72) Erfinder:
- **Tropsch, Jürgen, Dr.
D-67354 Römerberg (DE)**
- **Sanner, Axel, Dr.
D-67227 Frankenthal (DE)**
- **Hössel, Peter, Dr.
D-67105 Schifferstadt (DE)**
- **Raubenheimer, Hans-Jürgen
D-68775 Ketsch (DE)**
- **Schade, Christian, Dr.
D-67061 Ludwigshafen (DE)**

(56) Entgegenhaltungen:
**EP-A- 0 100 890**
**EP-A- 0 455 081**
**EP-A- 0 535 447**
**EP-A- 0 544 158**
**WO-A-94/06403**
**FR-A- 2 715 841**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 715 843 B1

**Beschreibung**

Die vorliegende Erfindung betrifft Polymere, erhältlich durch radikalisch initiierte Copolymerisation von Monomergemischen aus

(a) 5 bis 50 Gew.-% eines 1-Vinylimidazols oder eines quaternierten 1-Vinylimidazols,

(b) 20 bis 80 Gew.-% N-Vinylcaprolactam,

(c) 10 bis 60 Gew.-% N-Vinylpyrrolidon, und

(d) 0 bis 30 Gew.-% eines weiteren radikalisch copolymerisierbaren Monomeren, welches als Homopolymerisat eine Glasübergangstemperatur von mehr als 20°C aufweist,

und, sofern als Monomer (a) ein nicht quaternisiertes 1-Vinylimidazol eingesetzt wird, anschließende Quaterierung des Polymeren.

Weiterhin betrifft die Erfindung die Verwendung solcher Polymere als Wirkstoffe in kosmetischen Zubereitungen, bevorzugt in haarkosmetischen Zubereitungen, vor allem in Haarstylingmitteln mit Pflegeeigenschaften, sowie haarkosmetische Zubereitungen, enthaltend als Wirkstoff solche Polymere, insbesondere haarkosmetische Zubereitungen in Form von Schäumen.

Haarstylingmittel sollen der Haarfrisur nach Applikation einen starken und dauerhaften Halt vermitteln und zudem ein gutes Haargefühl (guter Griff, gutes Haarvolumen sowie geringe Klebrigkeit des Haares) verursachen, dabei statische Aufladung verhindern und konditionierend wirken, d.h., die Naßkämmbarkeit des Haares positiv beeinflussen.

Diese Vielzahl von Anforderungen wurde bisher nur durch die Kombination von anionischen bzw. neutralen Festigerpolymeren mit kationischen bzw. siliconhaltigen Konditioniermitteln oder Conditionerpolymeren erreicht.

In der WO 94/02115 sind Haarstylingmitteln in Form von Schäumen beschrieben, welche gleichzeitig festigend und konditionierend wirken sollen, und deshalb aus einer Mischung aus einem festigenden Polymer, einem polykationischen, konditionierendem Polymer und oberflächenaktiven Mitteln bestehen.

Aus der EP-A 544 158 ist die Verwendung von Homo- oder Copolymerisaten auf Basis von quaternierten 1-Vinylimidazolen als organische Polyelektrolyte in kosmetischen Zubereitungen bekannt.

Weiterhin sind aus der EP-A 455 081 nicht-quaternierte Copolymere aus 1-Vinylimidazol, N-Vinylcaprolactam und N-Vinylpyrrolidon bekannt, die sich vor allem für den Einsatz in Haarfestigungsmitteln eignen.

Aufgabe der vorliegenden Erfindung war es, verbesserte Polymere zu finden, die bei Verwendung in Haarstylingmitteln gleichzeitig festigende und pflegende Eigenschaften aufweisen.

Demgemäß wurden die eingangs definierten Polymere, deren Verwendung und haarkosmetische Zubereitungen, enthaltend solche Polymere, gefunden.

Die erfindungsgemäßen Copolymeren sind erhältlich durch radikalisch initiierte Copolymerisation von Monomerengemischen aus

(a) 5 bis 50 Gew.-%, vorzugsweise 7 bis 20 Gew.-% eines N-Vinylimidazols oder eines quaternierten N-Vinylimidazols,

(b) 20 bis 80 Gew.-%, vorzugsweise 40 bis 60 Gew.-% N-Vinylcaprolactam,

(c) 10 bis 60 Gew.-%, vorzugsweise 20 bis 50 Gew.-% N-Vinylpyrrolidon, und

(d) 0 bis 30 Gew.-%, vorzugsweise 0 bis 15 Gew.-% eines weiteren radikalisch copolymerisierbaren Monomeren, das als Homopolymerisat eine Glastemperatur von mehr als 20°C aufweist,

und, sofern als Monomer (a) ein nicht quaternisiertes N-Vinylimidazol eingesetzt wird, anschließende Quaternierung des Polymeren.

Geeignete N-Vinylimidazole (Monomere (a)) sind 1-Vinylimidazol-Derivate der allgemeinen Formel I,

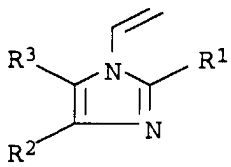

(I)

worin $R^1$ für Wasserstoff, $C_1$-$C_4$-Alkyl- oder Phenyl steht, $R^2$ und $R^3$ gleich oder verschieden sein können und für Wasserstoff oder $C_1$-$C_4$-Alkyl stehen.

Die Vinylimidazole können als freie Basen oder in quaternierter Form eingesetzt werden, wobei die Copolymerisation von quaternierten Vinylimidazolen bevorzugt ist. Setzt man die Vinylimidazole bei der Copolymerisation in Form der freien Basen ein, so erfolgt die Quaternierung nach der Polymerisation.

Zur Quaternisierung des Vinylimidazols eignen sich beispielsweise Alkylhalogenide mit 1 bis 22 C-Atomen in der Alkylgruppe, z. B. Methylchlorid, Methylbromid, Methyliodid, Ethylchlorid, Ethylbromid, Propylchlorid, Hexylchlorid, Dodecylchlorid, Laurylchlorid und Benzylhalogenide, insbesondere Benzylchlorid und Benzylbromid. Weitere geeignete Quaternierungsmittel sind Dialkylsulfate, insbesondere Dimethylsulfat oder Diethylsulfat. Die Quaternierung der Vinylimidazole kann auch mit Alkylenoxiden wie Ethylenoxid oder Propylenoxid in Gegenwart von Säuren durchgeführt werden. Bevorzugte Quaternierungsmittel sind: Methylchlorid, Dimethylsulfat oder Diethylsulfat.
Ganz besonders bevorzugte Monomere der Gruppe (a) sind 3-Methyl-l-vinylimidazolium-chlorid und -methylsulfat. Die Quaternierung der Monomeren oder eines Polymeren mit einem der genannten Quaternierungsmittel kann nach allgemein bekannten Methoden erfolgen.

Als Monomere (d) eignen sich beispielsweise $C_1$-$C_{12}$-Alkylester der Acrylsäure oder der Methacrylsäure wie tert.-Butylacrylat, Isobutylmethacrylat, n-Butylmethacrylat, Methylmethacrylat, Ethylmethacrylat, t-Butylmethacrylat, Isobornylacrylat oder Isobornylmethacrylat oder Acrylamide wie N-tert.-Butylacrylamid oder N-tert.-Octylacrylamid. Geeignet sind ferner Monomere, die in Wasser bei 25°C zu mehr als 5 Gew.-% löslich sind, zum Beispiel Acrylsäure, Methacrylsäure, Crotonsäure, N-Methylolmethacrylamid, N-Vinyl-N-methyl-acetamid, N-Vinylformamid, Acrylamid, N, N-Dimethylacrylamid, Methacrylamid, N-Vinyloxazolidon, N-Vinyltriazol, Hydroxyalkyl(meth)acrylate oder Alkylethylenglycol(meth)acrylate mit 1 - 50 Ethylenglycoleinheiten im Molekül.

Ganz besonders bevorzugt sind Copolymere aus

(a) 10 bis 30 Gew.-% 3-Methyl-1-vinyl-imidazoliummethylsulfat,

(b) 40 bis 60 Gew.-% N-Vinylcaprolactam, und

(c) 30 bis 50 Gew.-% N-Vinylpyrrolidon.

Die Herstellung der Polymerisate kann nach dem an sich bekannten Verfahren der radikalisch initiierten Polymerisation erfolgen. Vorzugsweise erfolgt die Herstellung als Lösungspolymerisation in Lösungsmitteln wie Wasser, Methanol, Ethanol, Isopropanol oder Mischungen dieser Lösungsmittel. Man wählt die Mengen an Monomeren und Lösungsmitteln zweckmäßigerweise so, daß 15 bis 60 gew.-%ige Lösungen entstehen. Die Polymerisation erfolgt üblicherweise bei Temperaturen von 60°C bis 130°C und bei Normaldruck oder unter Eigendruck.

Als Initiatoren für die radikalische Polymerisation können die hierfür üblichen Peroxo- und/oder Azo-Verbindungen eingesetzt werden, beispielsweise Dibenzoylperoxid, tert.-Butylperpivalat, tert.-Butyl-2-ethyl-hexanoat, Di-tert.-butylperoxid, tert.-Butylhydroperoxid, Azo-bis-isobutyronitril, Azo-bis-(2-amidinopropan)dihydrochlorid oder 2,2'-Azo-bis-(2-methylbutyronitril). Geeignet sind ebenfalls Initiatormischungen oder übliche Redoxinitiatoren. Die Initiatoren können in üblichen Mengen eingesetzt werden, beispielsweise 0,05 bis 5 Gew.-%, bezogen auf die Menge der zu polymerisierenden Monomere.

Das Molekulargewicht kann gewünschtenfalls durch den Zusatz von Reglern, beispielsweise Verbindungen, die Schwefel in gebundener Form enthalten, eingestellt werden.

Die K-Werte der Polymerisate sollen im Bereich von 10 bis 350, vorzugsweise 50 bis 300 liegen. Der jeweils gewünschte K-Wert läßt sich in an sich bekannter Weise durch die Wahl der Polymerisationsbedingungen, beispielsweise der Polymerisationsdauer und der Initiatorkonzentration und der Reglerkonzentration einstellen. Die K-Werte werden nach Fikentscher, Cellulosechemie, Bd. 13, S. 58-64 (1932) bei 25°C in 0,1 gew.-%iger wäßriger Lösung gemessen.

Die erfindungsgemäßen Polymere eignen sich zur Verwendung als Wirkstoffe in kosmetischen Zubereitungen, seien es hautkosmetische Zubereitungen wie Flüssigseifen, Körperlotionen, Rasierwasser, Gesichtswasser und anderen kosmetischen Lotionen oder, vor allem haarkosmetischen Zubereitungen wie Haarkuren, Haarlotionen, Haarspülungen, Haaremulsionen, Spitzenfluids, Egalisierungsmitteln für Dauerwellen, "Hot-Oil-Treatment"-Präparate, Conditioner, Festigerlotionen oder Haarsprays. Je nach Anwendungsgebiet können die haarkosmetischen Zubereitungen als Spray, Schaum, Gel, Gelspray oder Mousse appliziert werden.

Die haarkosmetischen Zubereitungen können neben den erfindungsgemäßen Polymeren und geeigneten Lösungsmitteln wie Wasser oder Wasser/Alkohol-Gemischen noch in der Kosmetik übliche Zusatzstoffe wie Emulgatoren, Konservierungsmittel, Parfümöle, Pflegestoffe wie Panthenol, Collagen, Vitamine, Eiweißhydrolysate, Stabilisatoren, pH-Wert-Regulatoren, Farbstoffe und weitere übliche Additive enthalten.

Die erfindungsgemäßen Polymerisate eignen sich insbesondere zur Verwendung in Haarstyling-Mitteln mit pflegenden Eigenschaften. Solche Haarstyling-Mittel können bevorzugt in Form von Schäumen wie Aerosolhaarschäumen oder Pumpschäumen angewandt werden oder als Haargele.

Erfindungsgemäße Haarstyling-Mittel werden vorzugsweise in Form von Schäumen angewandt. Solche für Schäume geeignete Zubereitungen können neben den Polymeren und Wasser oder Wasser/Alkohol-Gemischen als Lösungsmittel ebenfalls noch die in der Kosmetik üblichen Zusatzstoffe und übliche Additive enthalten. Für den Fall, daß die Haarstylingmittel als Aerosolschäume angewandt werden, enthalten sie übliche Treibmittel wie Gemische aus Propan/Butan, Dimethylether oder Druckluft. Sollen die Haarstyling-mittel als Pumpschäume angewandt werden, so erübrigt sich die Zugabe von Treibmitteln; die Applikation erfolgt dann mit den hierfür üblichen Vorrichtungen.

Eine erfindungsgemäß für die Aerosolschäume geeignete Zubereitung kann beispielsweise wie folgt zusammengesetzt sein:

(a1) 0,01 bis 10 Gew.-% eines erfindungsgemäßen Polymers,

(a2) 5 bis 20 Gew.-% eines Treibmittels, beispielsweise Propan/Butan,

(a3) 0,1 bis 5 Gew.-% eines Emulgators, beispielsweise Cetyldimethyl-2-hydroxyethylammoniumdihydrogenphosphat oder Cetheareth-25 (CTFA),

(a4) 0,1 bis 20 Gew.-% an üblichen kosmetischen Hilfsstoffen,

(a5) Wasser oder ein Wasser/Alkohol-Gemisch ad 100 Gew.-%.

Eine für Pumpschäume geeignete Zubereitung kann analog zusammengesetzt sein, jedoch ohne Treibmittel (a2).

Haarkosmetische Zubereitungen, die in Form von Gelen angewandt werden sollen, enthalten noch gelbildende Substanzen wie beispielsweise Carbomere (CTFA).

Man kann die erfindungsgemäßen Polymere auch mit herkömmlichen Haarkosmetik-Polymeren abmischen, falls ganz spezielle Eigenschaften eingestellt werden sollen.

Als herkömmliche Haarkosmetik-Polymere eignen sich beispielsweise anionische Polymere. Solche anionischen Polymere sind Homo- und Copolymere von Acrylsäure oder Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane und Polyharnstoffe. Besonders geeignete Polymere sind Copolymere aus t-Butylacrylat, Ethylacrylat, Methacrylsäure (z.B. Luvimer® 100P), Copolymere aus N-tert.Butylacrylamid, Ethylacrylat, Acrylsäure (Ultrahold® 8, strong), Copolymere aus Vinylacetat, Crotonsäure, Vinylpropiat (z.B. Luviset® CAP), Maleinsäureanhydridcopolymere, ggf. mit Alkoholen umgesetzt, anionische Polysiloxane, z.B. carboxyfunktionelle, Copolymere aus Vinylpyrrolidon, t-Butylacrylat, Methacrylsäure (z.B. Luviskol® VBM).

Ganz besonders bevorzugt werden als anionische Polymere Acrylate mit einer Säurezahl größer gleich 120 und Copolymere aus t-Butylacrylat, Ethylacrylat, Methacrylsäure.

Weitere geeignete Haarkosmetik-Polymere sind kationische Polymere wie Polyquaternium 1 bis x nach INCI, Copolymere aus Vinylpyrrolidon/Vinylimidazoliumsalzen (Luviquat® FC, Luviquat® HM) Copolymere aus Vinylpyrrolidon/Dimethylaminoethylmethacrylat, quaterniert mit Diethylsulfat (Luviquat® PQ 11); kationische Cellulosederivate (Polyquaternium-4 und 10) Acrylamidcopolymere (Polyquaternium-7). Besonders geeignet sind als kationische Polymere Copolymere aus Vinylpyrrolidon/Vinylimidazoliumchlorid 5:95 (Luviquat® FC 905).

Als weitere Haarkosmetik-Polymere sind auch neutrale Polymere geeignet wie Polyvinylpyrrolidone, Copolymere aus Vinylpyrrolidon und Vinylacetat und/oder Vinylpropionat, Polysiloxane, Polyvinylcaprolactam und Copolymere mit Vinylpyrrolidon, Polyethylenimine und deren Salze, Polyvinylamine und deren Salze, Cellulosederivate, Polyasparaginsäuresalze und Derivate. Bevorzugtes neutrales Polymeres ist Polyvinylcaprolactam.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane oder Silikonharze.

Haarkosmetische Zubereitungen, insbesondere Haarstylingmittel, die die erfindungsgemäßen Polymere als alleinige Wirkstoffe enthalten, weisen neben guten festigenden Eigenschaften auch gute pflegende Eigenschaften auf. Mit solchen Mitteln behandelte Haare vermitteln ein angenehmes Hautgefühl, sind nicht klebrig, gut kämmbar, statisch nicht aufgeladen. Das gute Filmbildevermögen der Polymere ermöglicht eine gute Verteilbarkeit auf dem Haar.

Herstellung der Copolymerisate

In einer Rührapparatur mit aufgesetztem Rückflußkühler wurden 300 g Wasser vorgelegt und unter Rühren im Stickstoffstrom auf 65°C erwärmt. Nach Erreichen dieser Temperatur wurde die Monomerenmischung (Zusammensetzung siehe Tabelle 1) in 250 g Wasser während 4 Stunden und ein Zulauf aus 2 g 2,2'-Azobis(2-amidinopropan) dihydrochlorid in 50 g Wasser während 5 Stunden zugegeben. Die Mischung wurde anschließend noch zwei Stunden bei dieser Temperatur gerührt. Man erhielt eine klare Lösung eines Polymerisats.

Tabelle 1

| Bsp. | Monomere (a) | N-Vinylcaprolactam (b) | N-Vinylpyrrolidon (c) | Monomere (d) | K-Wert |
|---|---|---|---|---|---|
| 1 | 3-Ethyl-1-vinylimidazoliummethylsulfat 80 g | 162 g | 162 g | - | 242 |
| 2 | 3-Methyl-1-vinylimidazoliumchlorid 40 g | 200 g | 160 g | - | 245 |
| 3 | 3-Methyl-1-vinylimidazoliummethylsulfat 40 g | 320 g | 40 g | - | 233 |
| 4 | 3-Methyl-1-vinylimidazolium-methylsulfat 40 g | 180 g | 160 g | tert.Butylacrylat 20 g | 236 |
| 5 | 3-Methyl-1-vinylimidazolium-methylsulfat 40 g | 300 g | 40 g | tert.Butylacrylat 20 g | 212 |
| 6 | 3-Methyl-1-vinylimidazolium-methylsulfat 40 g | 200 g | 160 g | - | 275 |
| 7 | 3-Methyl-1-vinylimidazolium-methylsulfat 120 g | 120 g | 160 g | - | 256 |

Herstellung von haarkosmetischen Formulierungen

| Formulierung 1: | |
|---|---|
| 03.00 g | Polymer gemäß Beispiel 3 |
| 00.10 g | Konservierungsmittel |
| 00.40 g | Parfümöl*) |
| 00.50 g | Cetyldimethyl-2-hydroxyethyl-ammonium-dihydrogenphosphat |
| 10.00 g | Propan/Butan |
| ad 100.00 g | Wasser |

*) Parfümöl als Mischung mit PEG-40-hydrogensulfated castor-oil (CTFA) im Gew.-Verhältnis 1:3

| Formulierung 2: | |
|---|---|
| 03.00 g | Polymer gemäß Beispiel 4 |
| 00.10 g | Konservierungsmittel |

(fortgesetzt)

| Formulierung 2: | |
|---|---|
| 00.40 g | Parfümöl*) |
| 00.50 g | Cetyldimethyl-2-hydroxyethyl-ammonium-dihydrogenphosphat |
| 10.00 g | Propan/Butan (1/3) |
| ad 100.00 g | Wasser |

*) Parfümöl als Mischung mit PEG-40-hydrogensulfated castor-oil (CTFA) im Gew.-Verhältnis 1:3

| Formulierung 3: | |
|---|---|
| 03.00 g | Polymer gemäß Beispiel 5 |
| 00.10 g | Konservierungsmittel |
| 00.40 g | Parfümöl*) |
| 00.50 g i | Cetyldimethyl-2-hydroxyethyl-ammonium-dihydrogenphosphat |
| 10.00 g | Propan/Butan (1/3) |
| ad 100.00 g | Wasser |

*) Parfümöl als Mischung mit PEG-40-hydrogensulfated castor-oil (CTFA) im Gew.-Verhältnis 1:3

| Formulierung 4: | |
|---|---|
| 03.00 g | Polymer gemäß Beispiel 2 |
| 00.10 g | Konservierungsmittel |
| 00.40 g | Parfümöl*) |
| 00.50 g | Cetyldimethyl-2-hydroxyethyl-ammonium-dihydrogenphosphat |
| 10.00 g | Propan/Butan (1/3) |
| ad 100.00 g | Wasser |

*) Parfümöl als Mischung mit PEG-40-hydrogensulfated castor-oil (CTFA) im Gew.-Verhältnis 1:3

| Formulierung 5: | |
|---|---|
| 03.00 g | Polymer gemäß Beispiel 6 |
| 00.10 g | Konservierungsmittel |
| 00.40 g | Parfümöl*) |
| 00.50 g | Cetyldimethyl-2-hydroxyethyl-ammonium-dihydrogenphosphat |
| 10.00 g | Propan/Butan (1/3) |
| ad 100.00 g | Wasser |

*) Parfümöl als Mischung mit PEG-40-hydrogensulfated castor-oil (CTFA) im Gew.-Verhältnis 1:3

| Formulierung 6 (Vergleichsbeispiel): | |
|---|---|
| 03.00 g | Polymer aus 50 Gew.-% N-Vinylpyrrolidon, 50 Gew.-% N-Vinylimidazoliumchlorid |
| 00.10 g | Konservierungsmittel |
| 00.40 g | Parfümöl*) |
| 00.50 g | Cetyldimethyl-2-hydroxyethyl-ammonium-dihydrogenphosphat |
| 10.00 g | Propan/Butan (1/3) |
| ad 100.00 g | Wasser |

*) Parfümöl als Mischung mit PEG-40-hydrogensulfated castor-oil (CTFA) im Gew.-Verhältnis 1:3

| Formulierung 7 (Vergleichsbeispiel): | |
|---|---|
| 01.00 g | Polymer (7a) aus 50 Gew.-% N-Vinylpyrrolidon/50 Gew.-% N-Vinylimidazoliumchlorid |
| 02.00 g | Polymer (7b) aus 60 Gew.-% N-Vinyl-pyrrolidon/40 Gew.-% Vinylacetat |
| 00.10 g | Konservierungsmittel |
| 00.40 g | Parfümöl*) |
| 00.50 g | Cetyldimethyl-2-hydroxyethyl-ammonium-dihydrogenphosphat |
| 10.00 g | Propan/Butan (1/3) |
| ad 100.00 g | Wasser |

*) Parfümöl als Mischung mit PEG-40-hydrogensulfated castor-oil (CTFA) im Gew.-Verhältnis 1:3

| Formulierung 8 (Vergleichsbeispiel) | |
|---|---|
| 01.20 g | Polymer (7a) |
| 03.30 g | Polymer (7b) |
| 00.10 g | Konservierungsmittel |
| 00.40 g | Parfümöl*) |
| 00.50 g | Cetyldimethyl-2-hydroxyethyl-ammonium-dihydrogenphosphat |
| 10.00 g | Propan/Butan (1/3) |
| ad 100.00 g | Wasser |

*) Parfümöl als Mischung mit PEG-40-hydrogensulfated castor-oil (CTFA) im Gew.-Verhältnis 1:3

| Formulierung 9 (Vergleichsbeispiel): | |
|---|---|
| 01.00 g | Polyquaternium-11 (CTFA) |
| 02.00 g | Polymer (7b) |
| 00.10 g | Konservierungsmittel |
| 00.40 g | Parfümöl*) |
| 00.50 g | Cetyldimethyl-2-hydroxyethyl-ammonium-dihydrogenphosphat |
| 10.00 g | Propan/Butan |
| ad 100.00 g | Wasser |

*) Parfümöl als Mischung mit PEG-40-hydrogensulfated castor-oil (CTFA) im Gew.-Verhältnis 1:3

| Formulierung 10 (Vergleichsbeispiel) | |
|---|---|
| 01.20 g | Polyquaternium-11 (CTFA) |
| 03.30 g | Polymer (7b) |
| 00.10 g | Konservierungsmittel |
| 00.40 g | Parfümöl*) |
| 00.50 g | Cetyldimethyl-2-hydroxyethyl-ammonium-dihydrogenphosphat |
| 10.00 g | Propan/Butan (1/3) |
| ad 100.00 g | Wasser |

*) Parfümöl als Mischung mit PEG-40-hydrogensulfated castor-oil (CTFA) im Gew.-Verhältnis 1:3

Beurteilung der Festigung am Modellkopf bei 65 % relativer Luftfeuchte

Es wurden 300 g Schaum hergestellt. Ein Modellkopf aus mitteleuropäischem Haar wird zweimal mit 14 gew.-%iger wäßriger Lösung von Natriumlaurylethersulfat gewaschen. Auf dem Kopf wurde ein Scheitel mit dem Kamm gezogen. Auf je eine Hälfte des Kopfes wird eine etwa tennisballgroße Menge eines erfindungsgemäßen Schaumes aufgetragen und gleichmäßig verteilt. Auf die andere Kopfhälfte noch jeweils ein Schaum gemäß eines Vergleichsbeispiels aufgetragen.

Ein zweiter Kopf wurde seitenvertauscht behandelt. Danach wurde mit separaten Kämmen von mindestens zwei Personen die Näßkämmbarkeit des Modellkopfes sowie die Verteilbarkeit des Schaumes auf dem Haar beurteilt (siehe unten). Über Nacht wurden die Köpfe bei Raumtemperatur getrocknet.

Am nächsten Tag beurteilten mindestens zwei Personen die Festigung, die Klebrigkeit und die statische Aufladung der Köpfe. Die Beurteilungen erfolgen nach der in Tabelle 2 angegebenen Notenskala.

Tabelle 2

| Beurteilungen: | Note 1 | Note 2 | Note 3 |
|---|---|---|---|
| Naßkämmbarkeit | sehr gut | gut | schlecht |
| Verteilbarkeit | sehr gut | gut | schlecht |
| Festigung | sehr gut | gut | schwach |
| Klebrigkeit | keine | schwach | stark |
| Aufladung | keine | schwach | stark |

Tabelle 3

| Beurteilung der Festigung | | | | | |
|---|---|---|---|---|---|
| Ergebnisse der Halbseitentests (Noten) | Formulierung 1 | Formulierung 2 | Formulierung 3 | Formulierung 4 | Formulierung 5 |
| Naßkämmbarkeit | 1 | 1 | 1 | 1 | 1 |
| Verteilbarkeit | 1 | 1 | 1 | 1 | 1 |
| Festigung | 1 | 1 | 1 | 1 | 1 |
| Klebrigkeit | 1 | 1 | 1 | 1 | 1 |
| Aufladung | 1 | 1 | 1 | 1 | 1 |
| Ergebnisse der Halbseitentests (Noten) | Formulierung 6 | Formulierung 7 | Formulierung 8 | Formulierung 9 | Formulierung 10 |
| Naßkämmbarkeit | 1 | 2 | 1 | 1 | 1 |
| Verteilbarkeit | 1 | 1 | 2 | 1 | 1 |
| Festigung | 3 | 3 | 2 | 2 | 2 |
| Klebrigkeit | 3 | 2 | 3 | 2 | 3 |
| Aufladung | 1 | 2 | 1 | 1 | 1 |

Bestimmung der Curl Retention

(nach Applikation aus Haarschaumlösung bei 75 % rel. Feuchte)

Formulierungen 1 bis 5 ohne Propan/Butan

Es wurden 180 g dieser Lösung hergestellt. Für die Bestimmung der Curl Retention werden Haarsträhnen von 2 g Gewicht und 15,5 cm Länge und aus mittelbraunem europäischen Menschenhaar verwendet.

Bestimmungsmethode

Die Haarsträhnen werden 1 Stunde in einer Ethanol/Wasser-Lösung (1:1) aufbewahrt, mit Wasser ausgespühlt und dann zweimal mit einer wäßrigen Texapon NSO-Lösung (ca. 0,5 % WS) gewaschen. Anschließend werden die Haarsträhnen mit Wasser bei ca. 40°C ausgespült bis keine Seifenbildung mehr erkennbar ist, gekämmt und in Wasser aufbewahrt.

Die feuchten Haare werden nun dreimal in die Polymerlösung (siehe oben) eingetaucht, zwischendurch mit den Fingern abgestreift und zwischen Filterpapier ausgedrückt. Danach wird das Haar um einen Teflonstab (12 mm Durchmesser) gewickelt und mit Filterpapier und Gummiring befestigt. Anschließend werden die Haarsträhnen 90 min bei 70°C im Wärmeschrank getrocknet.Nach Abkühlen auf Raumtemperatur werden die Locken abgestreift an einem eigens hierfür angefertigtem Plexiglasgestell aufgehängt und die Lockenlänge (L0) an der angebrachten Skala gemessen.
Für die Bestimmung eines Curl Retention Wertes sind 10 Haarlocken zu verwenden. Die Locken werden in eine Klimakammer mit 20°C und 75 % rel. Luftfeuchte gegeben. Ihre Längen (Lt) werden nach 5 Stunden gemessen.
Die Curl Retention errechnet sich wie folgt:

$$\text{Curl Retention in \%} = \frac{\text{L-Lt}}{\text{L-L0}} * 100$$

L = Länge der Haare (15,5 cm)
L0 = Länge der Haarlocke nach dem Trocknen
Lt = Länge der Haarlocke nach Klimabehandlung

Als Curl Retention wird der Mittelwert aus den 10 Einzelmessungen nach 5 h bei 20°C und 75 % rel. Feuchte angegeben. Ergebnisse sind in Tabelle 4 aufgelistet.

Tabelle 4

| | Formulierungen | | | | |
|---|---|---|---|---|---|
| | 1 | 2 | 3 | 4 | 5 |
| Curl Retention (%) | 90 | 84 | 98 | 87 | 87 |
| | Formulierungen | | | | |
| | 6 | 7 | 8 | 9 | 10 |
| Curl Retention (%) | 39 | 35 | 35 | 43 | 45 |

Bestimmung der Klebrigkeit

Die zu prüfenden Polymere wurden als 20 gew.-% ige wäßrige Lösung mit einem Rakel mit 120 µm Spaltbreite auf eine Glasplatte aufgebracht. Der Naßfilm wurde bei 75 % relativer Feuchte und 20°C über Nacht in einem Klimaschrank gelagert.
Auf die mit dem Polymer beschichtete Glasplatte wurde ein Plastic-Carbon-Band (Pelikan 2060, 50 mm breit) gelegt. Mit einem Gummistempel der Shore A-Härte 60 ± 5 wurde mit 250 N für 10 s belastet. Die Prüfung wurde im Klimaschrank bei 75 % rel. Feuchte durchgeführt.
In dem Maß, in dem die Polymeroberfläche klebrig ist, bleibt die Druckfarbe des Carbon-Bandes auf dem Polymerfilm haften (Beurteilung: Noten von 0 = nicht klebrig bis 5 = sehr stark klebrig, >5: Polymerfilm wird von der Glasplatte abgerissen).

| | Polymer bzw. Polymermischung aus Formulierung | | | | |
|---|---|---|---|---|---|
| | 1 | 2 | 3 | 4 | 5 |
| Klebrigkeit (Note) | 0-1 | 1 | 0 | 2 | 1-2 |
| | Polymer bzw. Polymermischung aus Formulierung | | | | |
| | 6 | 7 | 8 | 9 | 10 |
| Klebrigkeit (Note) | >5 | >5 | >5 | >5 | >5 |

Beispiel 8 bis 12

In den folgenden Beispielen sind Mischungen von Polymeren als Haarschäume formuliert. Die Zusammensetzung der Schäume sowie die Eigenschaften sind in Tabelle 5 aufgeführt.

Tabelle 5 - Haarschäume

Alle Rezepturbeispiele enthalten 10 Gew.-% Propan/Butan; 0,4 Gew.-% Parfumöl Carina/Cremophor RH 40; 0,1 Gew.-% Euxyl K 100

| Polymere/Beispiel Nr. Angaben in Gew.-% | 8 | 9 | 10 | 11 | 12 |
|---|---|---|---|---|---|
| Copolymer aus Vinylcaprolactam, Vinylpyrrolidon und Vinylimidazoliummethylsulfat 5:4:1 Gew. Anteile | 2 | 2 | 1 | 2 | 2 |
| Polyvinylcaprolactam | 1 | - | - | - | - |
| Copolymer aus Methacrylsäure, Ethylacrylat, tert.-Butylacrylat; 100 % neutralisiert mit Aminomethylpropanol (Luvimer 100 P) | - | 1 | 0,5 | - | - |
| Polyquaternium-16 mit 95 Gew.-% Vinylimidazoliumchlorid (Luviquat FC 905) | - | - | - | 0,5 | 0,5 |
| Cremophor A 25 | 0,1 | 0,1 | 0,1 | - | 0,1 |
| Luviquat Mono CP | - | - | - | 0,4 | - |
| Polyquaternium11 | - | - | - | - | - |
| Biegefestigkeit (cN) | 135 | 345 | 150 | 94 | 95 |
| Klebrigkeit (Note 1 bis 5) | 1 | 1 | 1-2 | 3 | 3 |

| Polymere/Beispiel Nr. Angaben in Gew.-% | 8 | 9 | 10 | 11 | 12 |
|---|---|---|---|---|---|
| Halbseitentest (Note 1 = sehr gut bis 3): | | | | | |
| Naßkämmbarkeit | 2-3 | 2 | 2-3 | 1 | 1 |
| Festigung | 1-2 | 1 | 2 | 2 | 2-3 |
| Glanz des trockenen Haares | 1 | 2 | 1-2 | 1-2 | 1-2 |

## Patentansprüche

1. Für die Verwendung in Haarstylingmitteln geeignete Polymere, erhältlich durch radikalisch initiierte Copolymerisation von Monomergemischen aus

   (a) 5 bis 50 Gew.-% eines 1-Vinylimidazols oder eines quaternierten 1-Vinylimidazols,

   (b) 20 bis 80 Gew.-% N-Vinylcaprolactam,

   (c) 10 bis 60 Gew.-% N-Vinylpyrrolidon, und

   (d) 0 bis 30 Gew.-% eines weiteren radikalisch copolymerisierbaren Monomeren, welches als Homopolymerisat eine Glasübergangstemperatur von mehr als 20°C aufweist,

   und, sofern als Monomer (a) ein nicht quaternisiertes 1-Vinylimidazol eingesetzt wird, anschließende Quaterierung des Polymeren.

2. Polymere nach Anspruch 1, enthaltend als Monomere (a) 3-Methyl-1-vinyl-imidazoliumchlorid oder -methylsulfat.

3. Polymere nach Anspruch 1, enthaltend als Monomere (a) 3-Ethyl-1-vinyl-imidazoliumethylsulfat.

4. Polymere nach Anspruch 1, erhältlich durch Copolymerisation eines Monomerengemisches aus

   (a) 10 bis 30 Gew.-% eines mit Dimethylsulfat oder Diethylsulfat quaternierten 1-Vinylimidazols,

   (b) 40 bis 60 Gew.-% N-Vinylcaprolactam, und

   (c) 30 bis 50 Gew.-% N-Vinylpyrrolidon.

5. Polymere nach einem der Ansprüche 1 bis 4, enthaltend als Monomer (d) tert.-Butylacrylat.

6. Verwendung von Polymeren gemäß einem der Ansprüche 1 bis 5 als Wirkstoffe in kosmetischen Zubereitungen.

7. Verwendung nach Anspruch 6 in haarkosmetischen Zubereitungen.

8. Verwendung von Polymeren nach Anspruch 6 oder 7 in Haarstyling-Mitteln mit Pflegeeigenschaften.

9. Verwendung nach Anspruch 6 in hautkosmetischen Zubereitungen.

10. Haarkosmetische Zubereitungen, enthaltend als Wirkstoffe Polymere gemäß einem der Ansprüche 1 bis 5.

11. Haarkosmetische Zubereitungen nach Anspruch 10, in Form von Aerosolschäumen.

12. Haarkosmetische Zubereitungen nach Anspruch 10, in Form von Pumpschäumen.

13. Haarkosmetische Zubereitung nach Anspruch 10, in Form eines Gels.

14. Haarkosmetische Zubereitungen nach einem der Ansprüche 10 bis 13, enthaltend neben den Wirkstoff-Polymeren noch weitere in der Haarkosmetik übliche Polymere.

15. Haarkosmetische Zubereitungen nach einem der Ansprüche 10 bis 14, enthaltend die in der Haarkosmetik üblichen Hilfsstoffe.

16. Hautkosmetische Zubereitungen, enthaltend als Wirkstoffe Polymere gemäß einem der Ansprüche 1 bis 5.

## Claims

1. A polymer suitable for use in hair styling compositions and - obtainable by free radical copolymerization of a monomer mixture comprising

    (a) from 5 to 50% by weight of a 1-vinylimidazole or of a quaternized 1-vinylimidazole,

    (b) from 20 to 80% by weight of N-vinylcaprolactam,

    (c) from 10 to 60% by weight of N-vinylpyrrolidone and

    (d) from 0 to 30% by weight of a further monomer which is capable of free radical copolymerization and, as a homopolymer, has a glass transition temperature of more than 20°C

    and, if an unquaternized 1-vinylimidazole is used as monomer (a), subsequent quaternization of the polymer.

2. A polymer as claimed in claim 1, containing 3-methyl-1-vinylimidazolium chloride or methylsulfate as monomer (a).

3. A polymer as claimed in claim 1, containing 3-ethyl-1-vinylimidazolium ethylsulfate as monomer (a).

4. A polymer as claimed in claim 1 obtainable by copolymerization of a monomer mixture comprising

    (a) from 10 to 30% by weight of a 1-vinylimidazole quaternized with dimethyl sulfate or diethyl sulfate,

    (b) from 40 to 60% by weight of N-vinylcaprolactam and

    (c) from 30 to 50% by weight of N-vinylpyrrolidone.

5. A polymer as claimed in any of claims 1 to 4, containing tert-butyl acrylate as monomer (d).

6. A method of using a polymer as claimed in any of claims 1 to 5 as an active ingredient in cosmetic formulations.

7. Use as claimed in claim 6 in cosmetic hair formulations.

8. A method of using a polymer as claimed in claim 6 or 7 in hair styling compositions having care properties.

9. Use as claimed in claim 6 in cosmetic skin formulations.

10. A cosmetic hair formulation containing a polymer as claimed in any of claims 1 to 5 as an active ingredient.

11. A cosmetic hair formulation as claimed in claim 10, in the form of an aerosol foam.

12. A cosmetic hair formulation as claimed in claim 10, in the form of a pump foam.

13. A cosmetic hair formulation as claimed in claim 10, in the form of a gel.

14. A cosmetic hair formulation as claimed in any of claims 10 to 13, containing, in addition to the polymer as an active ingredient, further polymers conventionally used in hair cosmetics.

15. A cosmetic hair formulation as claimed in any of claims 10 to 14, containing the assistants conventionally used in hair cosmetics.

16. A cosmetic skin formulation containing a polymer as claimed in any of claims 1 to 5 as an active ingredient.

## Revendications

1. Polymères appropriés pour l'utilisation dans des agents pour sculpture de la chevelure, pouvant être obtenus par

copolymérisation initiée par voie radicalaire de monomères de

(a) 5 à 50 % en poids d'un 1-vinylimidazole ou d'un 1-vinylimidazole quaternisé,
(b) 20 à 80 % en poids de N-vinylcaprolactame,
(c) 10 à 60 % en poids de N-vinylpyrrolidone, et
(d) 0 à 30 % en poids d'un autre monomère copolymérisable par voie radicalaire, qui présente à l'état d'homopolymère une température de transition vitreuse supérieure à 20°C,

et, dans la mesure où un 1-vinylimidazole non quaternisé est utilisé comme monomère (a), quaternisation subséquente du polymère.

**2.** Polymères selon la revendication 1, contenant comme monomère (a) du chlorure ou du méthylsulfate de 3-méthyl-1-vinyl-imidazolinium.

**3.** Polymères selon la revendication 1, contenant comme monomère (a) du méthylsulfate de 3-éthyl-1-vinylimidazolinium.

**4.** Polymères selon la revendication 1, pouvant être obtenus par copolymérisation d'un mélange de monomères de

(a) 10 à 30 % en poids d'un 1-vinylimidazole quaternisé avec du sulfate de diméthyle ou du sulfate de diéthyle,
(b) 40 à 60 % en poids de N-vinylcaprolactame,
(c) 30 à 50 % en poids de N-vinylpyrrolidone.

**5.** Polymères selon l'une des revendications 1 à 4, contenant comme monomère (d) de l'acrylate de tert-butyle.

**6.** Utilisation de polymères selon l'une des revendications 1 à 5 comme matières actives dans des préparations cosmétiques.

**7.** Utilisation selon la revendication 6 dans des préparations cosmétiques pour cheveux.

**8.** Utilisation de polymères selon la revendication 6 ou 7 dans des agents de sculpture de la chevelure ayant des propriétés d'entretien.

**9.** Utilisation selon la revendication 6 dans des préparations cosmétiques pour la peau.

**10.** Préparations cosmétiques capillaires, contenant comme matières actives des polymères selon l'une des revendications 1 à 5.

**11.** Préparations cosmétiques capillaires selon la revendication 10, sous forme de mousses aérosol.

**12.** Préparations cosmétiques capillaires selon la revendication 10, sous forme de mousses pour pompe.

**13.** Préparation cosmétique capillaire selon la revendication 10, sous forme d'un gel.

**14.** Préparations cosmétiques capillaires selon l'une des revendications 10 à 13, contenant, outre les polymères de matière active, encore d'autres polymères usuels en cosmétique capillaire.

**15.** Préparations cosmétiques capillaires selon l'une des revendications 10 à 14, contenant les adjuvants usuels en cosmétique capillaire.

**16.** Préparations cosmétiques pour la peau, contenant comme matières actives des polymères selon l'un des revendications 1 à 5.